# EUROPEAN PATENT SPECIFICATION

(11) **EP 3 010 496 B1**
(45) Date of publication and mention of the grant of the patent: **02.10.2019**
(21) Application number: 14741388.4
(22) Date of filing: 19.06.2014
(51) Int. Cl.: A61K 31/198, A61K 45/06, A61K 36/9066, A61K 31/05, A61K 31/12, A61K 31/593, A61P 19/02

(54) **ARGININE FOR USE IN THE TREATMENT AND/OR PREVENTION OF OSTEOARTHRITIS**
ARGININ ZUR VERWENDUNG BEI DER BEHANDLUNG UND/ODER PRÄVENTION VON OSTEOARTHRITIS
ARGININE UTILISABLE POUR TRAITER ET/OU PRÉVENIR L'ARTHROSE

(30) Priority: 20.06.2013 IT MI20131028
(43) Date of publication of application: 27.04.2016
(73) Proprietor: Italfarmaco SpA, 20126 Milano (IT)
(72) Inventor: COLOMBO, Giuseppe, I-20831 Seregno (MB) (IT); PASCALE, Walter, I-20080 Basiglio (MI) (IT)
(74) Representative: Pistolesi, Roberto
(86) International application number: PCT/IB2014/062450
(87) International publication number: WO 2014/203200

(56) References cited:
- EP-A1- 2 474 309
- WO-A1-2004/113311
- WO-A2-2006/138609
- CN-A- 101 780 181
- US-A1- 2006 122 147
- US-A1- 2008 287 349
- US-B1- 6 346 519
- PARK YOO-SIN ET AL: "Intra-articular injection of a nutritive mixture solution protects articular cartilage from osteoarthritic progression induced by anterior cruciate ligament transection in mature rabbits: a randomized controlled trial", ARTHRITIS RESEARCH AND THERAPY, BIOMED CENTRAL, LONDON, GB, vol. 9, no. 1, 26 January 2007 (2007-01-26), page R8, XP021026952, ISSN: 1478-6354, DOI: 10.1186/AR2114

## Description

### STATE OF THE ART

There are several diseases within the broad spectrum of disabling diseases in orthopaedics, including rheumatic diseases, osteoporosis, and arthrosis. Rheumatic diseases, which include diverse types of arthritis, such as rheumatoid arthritis, psoriatic arthritis, ankylosing spondylitis, and connectivitis in varying forms of involvement, are characterised by joint inflammation caused by a flow of antigen-antibody deposits at articular level with consequent alteration of the joint. Several treatments exist for managing rheumatic diseases, the most common of which include anti-inflammatory drugs (cortisone and NSAIDs) and monoclonal antibodies. A study exists which shows the use of arginine in rheumatoid arthritis specifically (Kwasny Krochin-B et al. Polskie Archiwum Medycyny Wewnetrznej (2012), 122 (6)).

Osteoporosis is a disease affecting the bones in which the skeleton undergoes loss of bone mass and resistance due to nutritional, metabolic, or pathological factors. The skeleton is therefore at greater risk of pathological fractures, following such decrease in bone density and changes in bone microarchitecture. Conventional pharmacological treatments, such as hormonal therapy and NSAIDs, also commonly include vitamin, mineral, and amino acid supplements, such as methionine and arginine. In particular, it is known that, by stimulating collagen production, arginine contributes to bone formation (Ammann, P., et al. Journal of Bone and MineralResearch, (2002) Volume 17, issue 7, p.1264-1272). Journal of Bone and MineralResearch, (2002) Volume 17, issue 7, p.1264-1272).

Unlike rheumatic diseases, and especially the various forms of arthritis originating from inflammatory processes and those which are characterised by inflammation at articular level, and osteoporosis, which is characterised by demineralisation of the bone structure, arthrosis (also known as osteoarthrosis or osteoarthritis) is a non-inflammatory multifactorial degenerative disease, which presents with cartilage thinning and alterations (D. Hughes, Surgery (2009), vol. 27: 2, pp. 75-79; Brandt K.D et al., MedClin North Am 93 (2009), pp. 1-24; Dunkley L. et al., Surgery (2012), 30: 2; pp. 67-71).

In particular, arthrosis is a disease which involves the diarthrodial joint, concerning all components thereof, i.e. synovial membrane, synovial fluid, cartilage, and bone, which leads to a degeneration process.

Arthrosis is therefore a disease which affects joints, in particular the joints most subject to wear, such as the lumbar vertebrae, the hip, the fingers, the toes and/or the knee due to the body weight load thereupon. The most common clinical symptoms and signs of the disease are mainly located in the joint concerned and include pain, limitation of movement, stiffness and/or joint deformity.

Arthrosis is one of the major causes of disability in the adult population worldwide as it affects approximately 10% of the general adult population and 50% of people aged over 60.

In paediatric subjects or in subjects of a young age, it can occur following post-traumatic or fracture sequelae.

During the onset of arthrosis, the formation of new connective tissue occurs with new bone tissue formed around the area concerned. The affected joint shows characteristic changes in cartilage, including thinning, fissures, and the formation of marginal osteophytes and areas of subchondral osteosclerosis in the areas subject to loads. The synovial membrane appears hyperaemic and hypertrophic, while the capsule is oedematous and fibrosclerotic. The onset of arthrosis or osteoarthrosis is characterised by a loss of balance between the cartilage degradation induction and inhibition mechanisms, and the chondrocyte thus activated leads to cartilage degeneration and, consequently, damage to the underlying bone.

The synovial membrane is a thin connective membrane which covers the joint capsule internally and the articular part of the bone. It is formed of a layer of endothelial cells located on a fibrous connective tissue; it also covers the intra-articular ligaments and the tendons. It contains the synovial fluid, i.e. a filamentous liquid, produced partly by the said membrane, which serves to lubricate the joints.

In subjects with arthrosis, this membrane becomes hypertrophic with the presence of inflammatory mediator cells.

The capsule is an envelope of dense connective tissue, which surrounds the connecting bony articular surfaces, covering the joint entirely. Its function is to protect the ends of the bones and keep them close (but not together). It is present in mobile joints.

In arthrosic subjects, the capsule undergoes inflammation.

Current management of arthrosis includes both non-pharmacological treatments, such as physical exercise, weight loss, physiotherapy, and gymnastics with kinesiological therapy, and pharmacological treatments, such as analgesics and non-steroidal anti-inflammatory drugs, and aims to act on the cartilage, relieving symptoms consisting mainly of pain and stiffness, and conserving and restoring the functions of the affected joints.

It is, however, known that current non-pharmacological treatments are, in most cases, insufficient to guarantee adequate treatment of the disease, providing only temporary relief from part of the symptomatology.

The disadvantages and side effects of these pharmacological treatments, however, are well known.

For example, treatments using analgesics and anti-inflammatory drugs are associated with the risk of gastric ulcer and duodenal ulcer, characterised by pain, burning, or nausea, ulceration of the gastric mucosa with possible haemorrhaging and possible skin reactions, such as erythema and urticaria, in susceptible individuals.

Apart from the anti-inflammatory/immunosuppressive effect, the side effects of the oral treatments using corticosteroids generally stem from the fact that they interfere with the body's homeostatic systems and therefore may cause: hypertension, water retention, hyperglycaemia, potassium loss, osteoporosis, muscle hypertrophy, capillary fragility, delayed wound healing, hyperlipidaemia, accumulation of adipose tissue in the face, neck and abdomen, gastroduodenal ulcers, increased blood coagulability, haematological disorders, euphoria, and insomnia.

Arthrosic patients are also often forced to undergo such pharmacological treatments for prolonged periods of time, if not for life, thereby exposing themselves to possible damage to other systems, such as the digestive system.

In the event of failure of such treatments, orthopaedic surgery is used nowadays, with the placement of implants or other orthopaedic devices, which involves known discomfort.

Over recent years, alongside the aforesaid pharmacological and non-pharmacological treatments, widespread use has also been made of chondroprotective supplements, such as glycosaminoglycans, glucosamine and/or chondroitin sulphate, which are known to be suitable substances for nourishing articular cartilage and activating production thereof. In particular, chondroprotectors can improve cartilage metabolism and therefore they are used with a view to delaying the progression of arthrosis by stimulating the synthesis of certain cartilage constituents and slowing down the cartilage tissue degeneration process in the affected joint.

Supplements available on the market include, for example, known formulations containing glycosaminoglycans (for example, chondroitin sulphate and/or glucosamine sulphate), with other additional components, such as types I and II collagen, diacerine, avocado extracts, and/or soy extracts (phytotherapy).

These products are well tolerated by patients, but have little effect on the symptoms and, in the stages characterised by acute pain, have to be combined with conventional drugs (such as NSAIDs).

Additional supplements today include known formulations containing glycosaminoglycans (for example, chondroitin sulphate and/or glucosamine sulphate) to which diverse active substances are added, such as amino acids (for example, methionine), vitamins (e.g. vitamin C, vitamin B6, vitamin B9, vitamin B12, vitamin D), minerals (zinc, copper) and/or fatty acids (for example DHA, EPA), which are involved in various ways in the regulation of the underlying processes of arthrosis (formulations whose trade names commonly contain prefixes or suffixes such as "*plus*" or "*forte*" (i.e. strong), e.g. Flexart plus, Fortilase, Reumilasesd).

Indeed, the role of methionine, for example, is known, since methionine is an amino acid which cannot be synthesised by the human body and is essential for activating the formation of cartilage due to its ability to act as a donor.

Furthermore, Document n. CN101780181A reports the possibility of using a composition comprising arginine and citrulline combined with other active ingredients (for example, vitamins, curcumin, folic acid, plant extracts, glucosamine sulphate, chondroitin sulphates, and other components) to treat osteoarthritis, using a high quantity of arginine (4-6 g). There is, therefore, a clear need for a new and improved approach to treating and preventing arthritis which has greater effectiveness and fewer side effects than the treatments used so far.

### DESCRIPTION OF THE INVENTION

It has surprisingly been found that arginine and/or citrulline play a significant role in the treatment and/or prevention of arthrosis.

The arginine and/or citrulline according to the present description represent a new and improved approach for the treatment and/or prevention of arthrosis, since both substances can act to improve bone metabolism, vascularise the bone, and activate repair of damage affecting the cartilage.

As can clearly be seen from the pre-clinical data illustrated in the experimental part, it has surprisingly been found that a composition comprising an aqueous arginine solution (administered to animals in daily dosages of approximately 20 and 50 mg) proves particularly effective in reducing and/or preventing the development of cartilage damage and ensuing bone damage in animal models of induced cartilage damage.

This effect has also been observed in compositions comprising a mixture of arginine and citrulline combined with other active ingredients (such as turmeric, vitamin D3 and resveratrol). This mixture was administered to animals starting from a minimum dose of 93 mg of the mixture, corresponding to 10.3 mg of arginine.

Overall, in the preclinical study, arginine was administered to rats in doses ranging from 10.3 to 51.6 mg. Based on the rat dose/human dose translation described by Reagan-Shaw S. et al. in "Dose translation from animal to human studies revisited", The FASEB Journal 22, 659-661 (2007), these quantities correspond to doses in humans ranging from 400 mg to 2000 mg.

The maximum dosage used is, therefore, effective in lower quantities than reported in the literature (CN101780181A).

One aim of the present invention is therefore to use arginine as the only active principle in the treatment and/or prevention of arthrosis, characterized in that the daily dose thereof ranges from 400 mg to 2000 mg.

The term "arthrosis" or "osteoarthrosis/osteoarthritis" according to the present invention refers to all classifications of arthrosis or osteoarthrosis, i.e. in the primary form (idiopathic, caused by genetic factors), the secondary form (caused by trauma, surgery, mechanical problems or septic problems), the localised form (monoarticular), and the generalised form (pluriarticular).

The term "arginine" according to the present invention refers to a nonessential amino acid (i.e. one that can be synthesised by the human body when other essential amino acids are supplied in a greater quantity than that required by the subject) which is involved in the synthesis of nitric oxide.

Arginine is a chiral molecule, i.e. a molecule which has a non-superposable three-dimensional mirror image.

The term "arginine" according to the present invention refers to both the "L" enantiomer and the "D" enantiomer of the molecule, and preferably the L enantiomer of arginine, as stated in Formula I below.

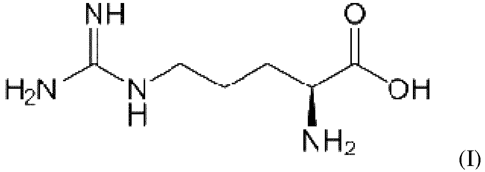

The term "citrulline" refers to a non-essential alpha amino acid (i.e. one that can be synthesised by the human body). Citrulline is an amino acid which is involved in the metabolic pathway of arginine and in nitric oxide synthesis.

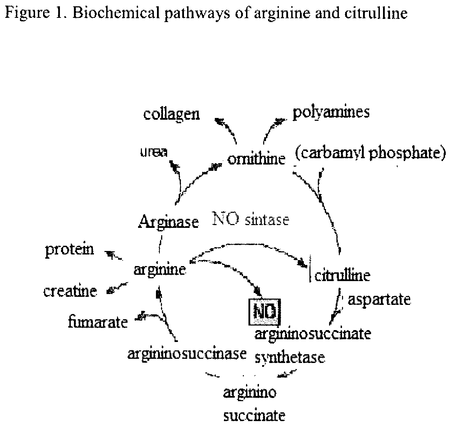

Citrulline is considered to be a metabolic precursor of arginine, since citrulline is transformed into arginine in the presence of aspartate and of the enzyme arginine succinate synthetase, according to the metabolic pathway shown in Figure 1.

Citrulline is also a chiral molecule, i.e. a molecule which has a non-superposable three-dimensional mirror image.

According to the present description, the term "citrulline" refers to both the "L" enantiomer and the "D" enantiomer of the molecule, and preferably the "L" enantiomer of citrulline, as stated in Formula II below.

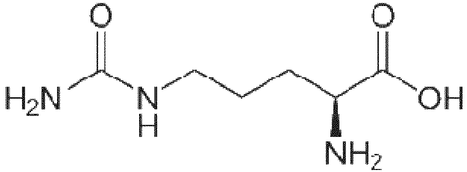

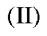

Arginine according to the present invention can be used in the forms described above or in the form of any pharmaceutically acceptable salt thereof. Preferably, the arginine is in the form of hydrochloride salt (arginine hydrochloride).

According to the present invention, the arginine can be administered orally or topically. When administered orally, the arginine according to the present invention may be in solid form or liquid form, and preferably in one of the following forms: tablet, capsule, granules, granulate, powder, pill, gel capsule, solution, suspension, emulsion, drops, syrup, powder for solutions, granules for solutions, powder for suspensions or granules for suspensions; more preferably in one of the following forms: tablet, capsule, granules, or powder.

When administered topically, the arginine according to the present invention may be in solid form, in semi-solid form, or in liquid form, and preferably in one of the following forms: cream, salve, ointment, gel, paste, milk, foam, medicated patch, lotion, powder for topical application, solution, suspension, emulsion; more preferably in one of the following forms: cream, salve, ointment, or gel.

According to the present description, the arginine may be administered in quantities ranging from 10 mg to 600 mg per dose, preferably from 10 mg to 400 mg per dose, and more preferably from 15 mg to 400 mg per dose, and even more preferably from 15 mg and 300 mg per dose, and still even more preferably in quantities of approximately 20 mg, approximately 75 mg, approximately 200 or 400 mg per dose. According to studies in the cardiovascular field, it has been seen that the administration of arginine in water at this dose kept the ARN/ADMA ratio close to healthy controls.

According to the present invention, the aforesaid dosages refer to arginine individually. According to one aspect of the present invention, the arginine can be administered as the sole active ingredient, combined with physiologically acceptable excipients. According to a further aspect of the present description, the citrulline can be administered as the sole active ingredient, combined with physiologically acceptable excipients.

According to a further aspect of the present description, a mixture of arginine and citrulline can be administered as the sole active ingredients, combined with physiologically acceptable excipients.

According to a further aspect of the present description, a mixture of arginine and citrulline can be administered with a further active ingredient, combined with physiologically acceptable excipients.

When mixed together, the arginine and the citrulline can be administered in equal quantities or in different quantities, although preferably in equal quantities. When administered mixed together, the preferable ratio can range from 1:5 to 1:1.

According to one embodiment of the present description, when administered orally, the arginine can be administered once a day, twice a day, or from one to four times a day, for a period of no less than 6 months, and more preferably once a day for 4 weeks, and even more preferably twice a day.

According to one embodiment of the present invention, the daily dosage of arginine ranges preferably from 400 mg to 1600 mg,

According to a further aspect of the present description, the arginine and/or citrulline can be administered either combined with or in close temporal proximity to at least one further active ingredient.

The said at least one further active ingredient according to the present description can therefore be administered together with the arginine and/or the citrulline (for example, contained in the same formulation), or it can be administered separately from the arginine and/or the citrulline (for example, contained in different compositions) or in temporal proximity with respect to the arginine and/or the citrulline (for example, contained in different compositions but administered in close temporal proximity).

The said at least one further useful active ingredient according to the present description should preferably be chosen from the following: amino acids, vitamins, fatty acids, glycosaminoglycans, turmeric, mangostin, *boswellia serrata,* resveratrol and/or a mixture thereof.

Useful amino acids according to the present description can be chosen from the following: methionine, S-adenosylmethionine and/or a mixture thereof. More preferably, S-adenosylmethionine.

Useful vitamins according to the present description can preferably be chosen from the following: vitamin B6, vitamin B9, vitamin B12, vitamin C, vitamin D, vitamin D3, vitamin K and/or a mixture thereof. More preferably, vitamin D3 and B-group vitamins, such as vitamin B6, vitamin B9 and/or vitamin B12.

Useful fatty acids according to the present description can be chosen from the following: omega-3 class fatty acids, such as docosahexaenoic acid (DHA) and eicosapentaenoic acid (EPA), omega-6 class fatty acids, omega-9 class fatty acids, and/or a mixture thereof; more preferably, omega 3 and/or omega-6 class fatty acids. Useful glycosaminoglycans according to the present description can preferably be chosen from the following: glucosamine sulphate, chondroitin sulphate, type II collagen, and/or a mixture thereof.

Useful curcuma according to the present description is preferably in the form of dry extract titrated in curcuminoids, due to the anti-inflammatory action thereof, as demonstrated in several clinical studies, including studies on osteo-articular disease (B. Chandran et al. A Randomised, Pilot Study to Assess the Efficacy and Safety of Curcumin in Patients with Active Rheumatoid Arthritis. Phytother Res. 2012 Mar 9. doi: 10.1002/ptr.4639).

Resveratrol is useful according to the present description due to the beneficial effects of its antioxidant activity on BMD (bone mineral density) and the risk of fractures, as demonstrated in several clinical studies (Mukamal KJ et al. Alcohol consumption, bone density, and hip fracture among older adults: the cardiovascular health study. Osteoporos Int. 18(5):593-602, 2007; Tucker K.L. et al.Effects of beer, wine, and liquor intakes on bone mineral density in older men and women. Am. J.Clin.Nutr. 89(4):1188-96, 2009; Yin J. et al. Beverage-specific alcohol intake and bone loss in older men and women: a longitudinal study.Eur. J. ClinNutr. 2011 Feb. 16).

The said at least one further active ingredient according to the present description can be administered in a variable quantity depending on the further active ingredient used. Preferably, the said at least one further active ingredient can be administered in a quantity weighing from 0.001 mg to 2100 mg per dose, preferably from 0.01 mg to 1600 mg per dose.

According to a preferred aspect, the S-adenosylmethionine is administered in a quantity weighing from 100 mg to 800 mg per dose, more preferably from 200 mg to 400 mg per dose, and even more preferably approximately 250 mg.

According to a further preferred aspect, the vitamin B6 is administered in a quantity weighing from 0.5 mg to 5 mg per dose, preferably from 1 mg to 3 mg per dose, and more preferably at a rate of approximately 1.4 mg per dose.

According to a still further preferred aspect, the vitamin D3 is administered in a quantity weighing from 0.001 mg to 0.005 mg per dose, preferably from 0.002 mg to 0.003 mg per dose, and more preferably approximately 0.0025 mg per dose.

According to a still further preferred aspect, the vitamin B9 is administered in a quantity weighing from 0.100 mg to 0.400 mg per dose, more preferably approximately 0.200 mg per dose.

According to a still further preferred aspect, the vitamin B12 is administered in a quantity weighing from 0.001 mg to 0.005 mg per dose, more preferably approximately 0.0025 mg per dose.

According to a still further preferred aspect, the DHA fatty acid is administered in a quantity weighing from 100 mg to 1000 mg per dose, preferably from 150 mg to 500 mg per dose, and more preferably approximately 200 mg per dose.

According to a still further preferred aspect, the EPA fatty acid in the aforesaid composition is administered in a quantity weighing from 10 mg to 200 mg per dose, preferably from 20 mg to 100 mg per dose, and even more preferably approximately 40 mg per dose.

According to a still further preferred aspect, the glucosamine sulphate contained in the aforesaid composition is administered in a quantity weighing from 250 mg to 2000 mg per dose, preferably from 750 mg to 1800 mg per dose, and even more preferably approximately 1500 mg per dose.

According to a still further preferred aspect, the chondroitin sulphate is administered in a quantity weighing from 500 mg to 1800 mg per dose, preferably from 800 mg to 1500 mg per dose, and more preferably approximately 1200 mg per dose.

The arginine according to the present invention is preferably administered to humans, intending thereby both adults and the "paediatric population", where the term "paediatric population" refers to the part of the population ranging from birth to eighteen years of age.

One advantage of the use of arginine according to the present invention is therefore the improved treatment and/or prevention of arthrosis (or osteoarthrosis). This improved treatment and/or prevention is due first and foremost to the ability of the substance(s) to act at bone level, improving bone metabolism and vascularisation. Secondly, the arginine according to the invention also act on the activation of the repair of damage affecting the cartilage, thereby contributing to the aforesaid action at bone level to complete the treatment and/or prevention of the disease.

A further aspect of the present description is to provide a pharmaceutical and/or food composition (for example, a supplement) and/or a medical device containing arginine and/or citrulline, and optionally at least one pharmaceutically or physiologically acceptable excipient and/or adjuvant.

The said at least one pharmaceutically or physiologically acceptable excipient and/or adjuvant according to the present description can be chosen from among any excipient and/or adjuvant which is useful and/or suitable for the formulation of pharmaceutical and/or food compositions and/or medical devices.

Preferably, the said at least one excipient and/or adjuvant can be chosen from the following: diluents, glidants, binders, lubricants, disintegrants, emulsifiers, anti-agglomerants, stabilisers, coating agents, flavourings, sweeteners, acidifiers, preservatives, solubilisers, humectants, viscosifiers, suspending agents, surfactants, wetting agents, buffers, acidifying agents, basifying agents, antioxidants, chelating agents, colourants, complexing agents, emollients, odour-improving agents (perfumes), thickening agents, agents for improving intestinal absorption and/or a mixture thereof.

More preferably, they can be selected from the following: stabilisers, disintegrants, lubricants, coating agents, binders, preservatives, diluents and agents for improving intestinal absorption and/or a mixture thereof. The said pharmaceutical and/or food composition can be formulated in a solid, semi-solid or liquid form.

When formulated in a solid form, the composition according to the present description is preferably in one of the following forms: tablet, capsule, granules, granulate, powder, pill, gel capsule, solution, suspension, emulsion, drop, syrup, powder for solutions, granules for solutions, powder for suspensions or granules for suspensions. More preferably, the composition is in one of the following forms: tablet, capsule, granules, granulate, or powder.

Optionally, the solid forms referred to above can be formulated in a gastro-resistant form, preferably comprising a gastro-resistant coating.

When in tablet form, the composition can be in any of the following forms: swallowable, effervescent, soluble, dispersible, orodispersible and/or gastro-resistant. The tablet according to the present description may also, optionally, be of the modified- or controlled-release variety.

The term 'modified-release' or 'controlled-release' refers to a formulation capable of releasing the active ingredient according to planned concentrations and kinetics.

When in capsule form, the composition according to the present description can take any of the following forms: hard capsule, soft capsule, gastro-resistant capsule, modified- or controlled-release tablet or capsule.

When in granule, granulate, or powder form, the composition according to the present description can take any of the following forms: effervescent, soluble, dispersible, gastro-resistant, although preferably the gastro-resistant form, contained in a sachet. The granules, granulate, or powder according to the description may also, optionally, be of the modified- or controlled-release variety.

The solid forms stated above can also be formulated in, or contain, at least one enterosome.

The term "enterosome" according to the present description refers to a delivery system capable of modulating the breakdown of the solid form, and the consequent release of the active ingredient, at intestinal level.

In particular, the enterosome is included or contained within the solid form, which is preferably a tablet, capsule, or granulate, where it constitutes a core within which, in turn, at least one active ingredient and at least one excipient and/or adjuvant are contained. The said excipient and/or adjuvant are present in the core in order to modulate the release of the active ingredient within the intestine, thereby improving absorption thereof through the intestinal epithelium.

This core can be also coated with a gastro-resistant coating which guarantees the arrival thereof intact at intestinal level.

When formulated in semi-solid form, the composition according to the present description is preferably in one of the following forms: cream, salve, ointment, gel, paste, milk, foam, medicated patch, lotion, powder for topical application, solution, suspension, or emulsion; more preferably in one of the following forms: cream, salve, ointment, or gel.

When formulated in liquid form, the composition according to the present description is preferably in one of the following forms: solution, suspension or emulsion.

The arginine and/or citrulline is contained in the pharmaceutical and/or food composition according to the present description in a quantity weighing from 10 mg to 600 mg, preferably from 10 mg to 400 mg, more preferably from 15 mg to 400 mg, even more preferably from 15 mg to 300 mg, and even more preferably approximately 20 mg, approximately 75 mg, or approximately 200 or 400 mg.

According to the present description, the aforesaid dosages refer to the arginine or citrulline in the composition individually.

According to one aspect of the present description , the arginine contained in the pharmaceutical and/or food composition can be administered as the sole active ingredient, combined with physiologically acceptable excipients.

According to a further aspect of the present description, the citrulline contained in the composition can be administered as the sole active ingredient, combined with physiologically acceptable excipients.

According to a further aspect of the present description, the mixture of arginine and citrulline present in the composition can be administered as the sole active ingredients, combined with physiologically acceptable excipients.

According to a further aspect of the present description, the mixture of arginine and citrulline can be present in the composition with a further active ingredient, combined with physiologically acceptable excipients.

When mixed together, the arginine and the citrulline can be contained in the composition in equal quantities or in different quantities, although preferably in equal quantities.

The pharmaceutical and/or food composition according to the present description can also contain at least one further active ingredient. The said at least one further active ingredient can be chosen from the following: amino acids, vitamins, fatty acids, glycosaminoglycans, curcuma, *boswellia serrata,* resveratrol, mangostin, and/or a mixture thereof.

Useful amino acids according to the present description can be chosen from the following: methionine, S-adenosylmethionine and/or a mixture thereof.

Useful vitamins according to the present descriptioncan preferably be chosen from the following: vitamin B6, vitamin B9, vitamin B12, vitamin C, vitamin D, vitamin D3, vitamin K, and/or a mixture thereof.

Useful fatty acids according to the present description are preferably chosen from the following: omega-3 class fatty acids, such as docosahexaenoic acid (DHA) and eicosapentaenoic acid (EPA), omega-6 class fatty acids, omega-9 class fatty acids, and/or a mixture thereof.

Useful glycosaminoglycans according to the present description can preferably be chosen from the following: glucosamine sulphate, glycosaminoglycan, chondroitin sulphate, and/or a mixture thereof.

Preferably, the said at least one further active ingredient can be contained in the composition according to the present description in a quantity weighing from 0.001 mg to 2100 mg per dose, preferably from 0.01 mg to 1600 mg per dose.

According to a preferred aspect of the description, the S-adenosylmethionine is contained in the composition in a quantity weighing from 200 mg to 800 mg per dose, more preferably from 200 mg to 400 mg per dose, and even more preferably approximately 250 mg.

According to a further preferred aspect of the description, the vitamin B6 is contained in the composition in a quantity weighing from 0.5 mg to 5 mg per dose, more preferably from 1 mg to 3 mg per dose, and even more preferably approximately 1.4 mg. According to a still further aspect of the description, the vitamin D3 is contained in the composition in a quantity weighing from 0.001 mg to 0.005 mg per dose, more preferably from 0.002 mg to 0.003 mg per dose, and even more preferably approximately 0.0025 mg.

According to a still further aspect of the description, the vitamin B9 is contained in the composition in a quantity weighing from 0.100 mg to 0.400 mg per dose, more preferably approximately 0.200 mg.

According to a still further aspect of the description, the vitamin B12 is contained in the composition in a quantity weighing from 0.001 mg to 0.005 mg per dose, more preferably approximately 0.0025 mg.

According to a still further aspect of the description, the DHA fatty acid is contained in the composition in a quantity weighing from 100 mg to 1000 mg per dose, preferably from 150 mg to 500 mg, and more preferably approximately 200 mg.

According to a still further aspect of the description, the EPA fatty acid is contained in the composition in a quantity weighing from 10 mg to 200 mg per dose, preferably from 20 mg to 100 mg, and more preferably approximately 40 mg, with respect to the total weight of the composition.

According to a still further aspect of the description, the glucosamine sulphate is contained in the composition in a quantity weighing from 250 mg to 2000 mg per dose, preferably from 750 mg to 1800 mg, and more preferably approximately 1500 mg, with respect to the total weight of the composition.

According to a still further aspect of the description, the chondroitin sulphate is contained in the composition in a quantity weighing from 500 mg to 1800 mg per dose, preferably from 800 mg to 1500 mg, and more preferably approximately 1200 mg, with respect to the total weight of the composition.

According to the description, the pharmaceutical or food composition according to the present invention can be administered to humans, intending thereby both adults and the "paediatric population", where the term "paediatric population" refers to the part of the population ranging from birth to eighteen years of age.

A further aspect of the present description is to provide a pharmaceutical or food composition, as described in detail above, for use in the treatment and/or prevention of arthrosis, as stated in detail above.

The following examples are intended merely to allow better understanding of the invention without in any way limiting it.

### DESCRIPTION OF THE FIGURES

Figure 1. Biochemical pathways of arginine and citrulline
Figure 2. Histological evaluation of the effect of the test compounds on cartilage damage (A), bone resorption (B), "pannus" formation (C) and reactive cell infiltration (D).
Figure 3. Histological evaluation of the effect of the compositions under examination expressed as a Total Histological Score.
Figure 4. Radiological evaluation of the effect of the compositions under examination on bone erosion (A), articular erosion (B), intra-articular space (C) and limb oedema (D).
Figure 5. Radiological evaluation of the effect of the compositions under examination expressed as a Total Radiological Score.

### Experimental part

### EXAMPLES

### Example 1: The pharmaceutical or food composition in the form of a gastro-resistant tablet

- Citrulline 200 mg
- Arginine 75 mg
- Vitamin D3 0.0025 mg
- Resveratrol 15 mg
- Curcuma 225 mg
- -Excipients: quantity as required

### Example 2: Pharmaceutical or food composition in gastro-resistant granulate form, in sachets:

- Arginine 400 mg
- Citrulline 200 mg
- Curcuma 225 mg
- Vitamin D3 0.0025 mg
- Resveratrol 10 mg
- Excipients: quantity as required

### Example 3. Analysis of oxidative stress markers in patients with arthrosis.

### Definitions

The term "MAD" according to the present invention refers to malondialdehyde, a reactive aldehyde produced by the peroxidation of polyunsaturated fatty acids which acts as an oxidative stress marker.

The term "ARN" according to the present invention refers to arginine neutral, which is involved in the production of nitric oxide.

The term "ADMA" according to the present invention refers to asymmetric dimethylarginine, a natural chemical which is found in blood plasma and whose function is to interfere with the arginine in nitric oxide synthesis.

The term "SDMA" according to the present invention refers to symmetric dimethylarginine, a natural chemical which acts on the inhibition pathway of nitric oxide.

### Materials and methods

Subjects selected comprised 32 patients with idiopathic arthrosis with prosthetic knee replacement surgery planned and 32 healthy control subjects.

A 3 ml sample of peripheral blood and 3 ml sample of synovial fluid were taken from both the patients with arthrosis and the corresponding healthy control subjects.

The following markers, which are used to show arthrosis severity and/or progression, were assayed using a HPLC (High Performance Liquid Chromatography) in order to establish levels thereof in the blood and synovial fluid.

### Results

**Table 1. Concentration of oxidative stress markers in plasma of arthrosic patients and control subjects.**

| **Oxidative stress markers** | **Mean concentration in patients (µM)** | **St. Dev.** | **Mean concentration in control subjects (µM)** | **St. Dev.** | **p** |
|---|---|---|---|---|---|
| **MDA** | 1.82 | 1.42 | 0.55 | 0.12 | P<0.001 |
| **ARN** | 53.55 | 16.73 | 70.20 | 25.68 | P<0.05 |
| **ADMA** | 0.69 | 0.14 | 0.64 | 0.14 | N.S. |
| **SDMA** | 0.69 | 0.15 | 0.61 | 0.10 | P<0.05 |
| **ARN/ADMA** | 80.85 | 29.58 | 105.57 | 30.48 | P<0.05 |

The results given in Table 1 show that the MDA concentration is greater in the patients' plasma than in the control subjects' (1.82 as against 0.55 µM, p<0.001), the ARN concentration is lower in the patients' plasma (53.55 as against 70.06 µM, p<0.05), the SDMA concentration is higher in the patients' plasma (0.69 as against 0.61 µM, p<0.05), while the ARN/ADMA ratio is lower in the patients than the control subjects. This means there is more arginine in the healthy subjects than in the arthrosic patients, for whom the arginine must be supplemented.

**Table 2. Concentration of oxidative stress markers in the plasma and synovial fluid of arthrosic patients**

| **Oxidative stress markers** | **Mean concentration in the plasma (µM)** | **St. Dev.** | **Mean concentration in the synovial fluid (µM)** | **St. Dev.** | **p** |
|---|---|---|---|---|---|
| **MDA** | 1.82 | 1.42 | 0.74 | 0.26 | P<0.0001 |
| **ARN** | 53.55 | 16.73 | 76.96 | 6.83 | P<0.00001 |
| **ADMA** | 0.69 | 0.14 | 0.75 | 0.09 | P<0.05 |
| **SDMA** | 0.69 | 0.15 | 0.66 | 0.10 | N.S. |
| **ARN/ADMA** | 80.85 | 29.58 | 103.37 | 13.65 | P<0.0005 |

The results given in Table 2 show that the MDA concentration in the patients' synovial fluid is less than the concentration in the plasma (0.74 as against 1.82, p <0.0001), the ARN concentration is greater (76.96 as against 53.55, p <0.00001), while the ARN/ADMA ratio is higher in the synovial fluid than in the plasma (103.37 as against 80.85 p <0.0005).

The lesser quantity of ARN in the patients' plasma explains the poor availability of nitric oxide (NO), which is related to the environment being more susceptible to oxidative stress and having greater wall rigidity.

The results obtained demonstrate, for the first time, how two molecules which are strongly related to oxidative stress, such as asymmetric dimethylarginine (ADMA) and the malondialdehyde (MDA), increase in the synovial fluid and blood of patients suffering from arthrosis.

The results therefore demonstrate, for the first time, a close correlation between arthrosis, oxidative stress, and vascular damage affecting cartilage in joints. It therefore shows how reactive oxygen species, which are responsible for oxidative stress, play an active role in the cartilage alteration process in joints, and likewise an important role in the onset and progression of arthrosis.

Since the arginine and/or citrulline according to the present invention are precursors of nitric oxide, known to be a potent vasodilator, the treatment according to the present invention allows action on the vascularisation of bone and articular cartilage, with the surprising advantage that it restores the relaxation potential of the smooth muscle of the endothelium of subchondral vessels, which cause oxidative stress and the resulting arthrosic damage.

### Example 4. Evaluation of the effectiveness of two compositions, following chronic oral administration, in an animal model of collagen-induced cartilage damage in Lewis rats.

### Introduction

The effectiveness of the two compositions under examination in reducing and/or preventing development of cartilage damage and resulting bone damage was assessed in an animal model using Lewis rats.

This model is based on the administration of heterologous type II collagen emulsified in Incomplete Freund's Adjuvant. Following this treatment, the appearance of polyarticular erosive damage was observed at both cartilaginous level and bone level, which reached maximum severity level after approximately 30 days.

The effectiveness of pharmacological treatments aimed at reducing osteocartilaginous damage in the affected limbs was then evaluated both radiographically and histologically at the end of the experimental period.

### Materials and methods

The study was conducted in a test facility issued with a certificate of compliance with Good Laboratory Practices and in accordance with Italian Legislative Decree No. 116/92 (27.01.92) on animal experimentation.

### Induction of disease

36 male Lewis rats (Harlan Italy) aged between 7 and 10 weeks were used. After an acclimatisation period of at least 4 days, the disease was induced (day 0) by intradermal injection (at the base of the tail) of 400 micrograms of type II bovine collagen (BCII) in a volume of 0.2 mL of Incomplete Freund's Adjuvant (IFA) acidified with acetic acid.

All tail base intradermal injections were carried out on animals anesthetised with isoflurane. After eight days (day 7), a second dose of BCII (100 g) was administered in a volume of 0.05 mL.

At both experimental times, the control animals (naive) were treated with acidified IFA only.

On the 29^{th} day, radiographic analysis was performed on the hind legs while histological analysis was performed on one limb only.

The radiological analysis was performed "blind" and involved assignment of a Radiological Score (RS), which is defined as follows:
- SIA - alterations in intra-articular space
- EA - articular erosion
- EO - bone erosion
- Etot, osteoarticular erosion (sum of EA and EO)
- RS total, the sum of all the other radiological scores

The score, which ranges from 0 (normal) to 4 (full-blown damage), is the sum of the scores (0 or 1, i.e. present or absent) obtained based on the observation of the four targets (oedema, articular erosion, bone erosion, and intra-articular space).

The histological analysis was performed "blind" and involved assignment of a Histological Score (HS), which is defined as follows:
- Infiltration of inflammatory cells
- Presence of "pannus"
- Cartilage damage
- Bone resorption

Where 'pannus' is the layer of fibrotic and granulation tissue, comprising fibroblasts and inflammatory cells, which forms in the joint and causes the destruction of cartilage and bone.

Each of the four disease targets contributes to the total score with its own score ranging from 0 (normal) to 3 (severe), through light and noticeable (1 and 2 respectively).

Since the development of the disease can vary from subject to subject, the data analysis was performed on 4 animals/group, after excluding those animals within each treatment group which showed different growth rates from the average growth of the respective group.

More specifically, the two rats in each group that showed, respectively, the lowest and the highest percentage of BW increase between day 0 and day 14 were excluded from further analysis.

### Compositions used:

### Composition A

| | |
|---|---|
| Curcuma rhizome titrated in curcuminoids | 225.0 mg |
| Vitamin D3 | equal to 2.5 mcg 50% RDA |
| Resveratrol | 10.0 mg |
| Arginine | 400.0 mg |
| Citrulline | 400.0 mg |
| Excipients | Quantity as required at 3600 mg |

### Composition B

Arginine, 30% solution in water.

### Treatment of the animals

Composition A (powder) was administered resuspended in carboxymethyl cellulose (CMC), while Composition B (30% solution) was diluted in water in the desired dose. Both compositions were administered orally (1 ml/rat) from day 1 to day 28 by gavage. After the acclimation period, the animals were randomly divided into 6 experimental groups (n = 6) according to the chart below.
Group V N-CIA: CMC (Animals 1, 2 and 3), water (Animals 4, 5 and 6)
Group V: CMC (Animals 1, 2 and 3), water (Animals 4, 5 and 6)
Group A1: Composition A, 93 mg/rat
Group A2: Composition A, 187 mg/rat
Group A3: Composition A, 516 mg/rat
Group B1: Composition B, 70 µL/rat (corresponding to 21 mg/rat)
Group B2: Composition B, 172 µL/rat (corresponding to 51.6 mg/rat)

The disease was induced in all experimental groups with the exception of group V N-CIA, which constitutes the control group (treatment-naive animals).

### Results

The effect of daily treatment with the compositions under examination has been evaluated both histologically and radiologically.

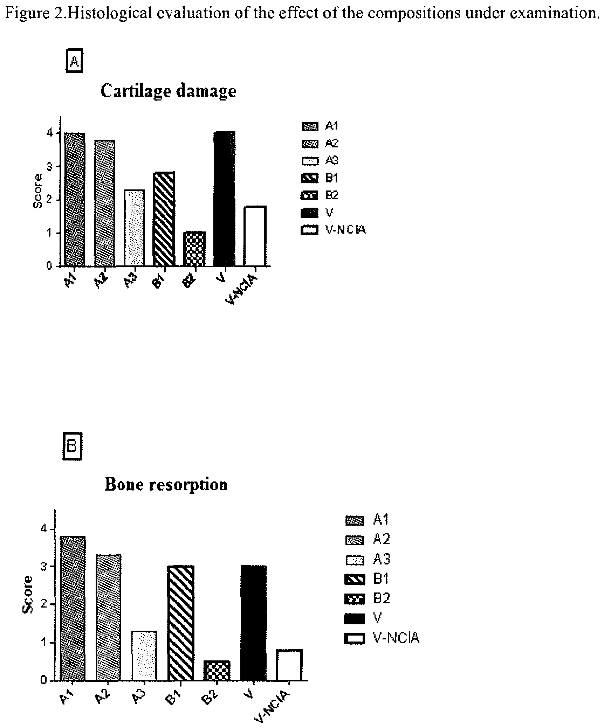

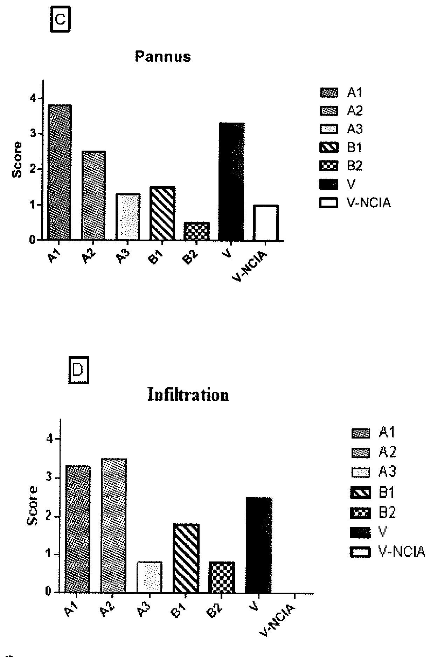

The histological analysis results are shown in Figure 2.

As regards cartilage damage (Figure 2A), the results obtained show that Composition B (Group B2) is effective in reducing damage in a dose-dependent manner.

At the higher dose (Group A3), Composition A resulted active, inhibiting development of cartilage damage by approximately 50%.

Bone resorption (Figure 2B) was completely normalised in the Group B2 animals and strongly reduced in the Group A3 animals.

The formation of "pannus" (Figure 2C) and the presence of infiltrate (Figure 2D) were significantly reduced by both compositions administered in the higher doses (Group B12, B2, A2 and A3).

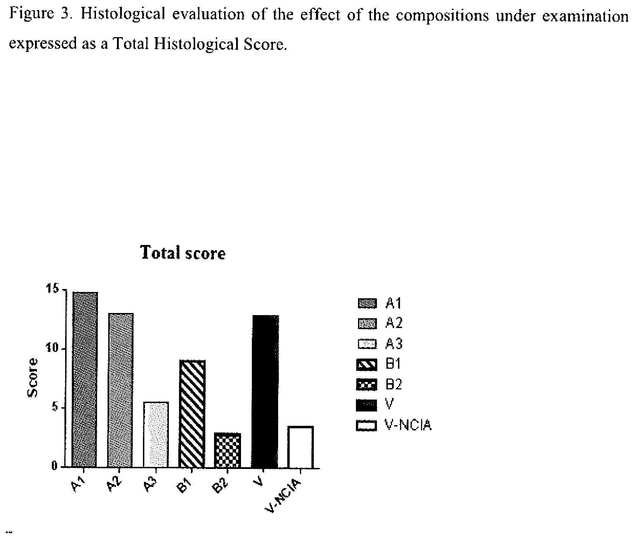

The greater effectiveness of Composition B with respect to Composition A is clear when the Histology Total Score is analysed, as shown in Figure 3.

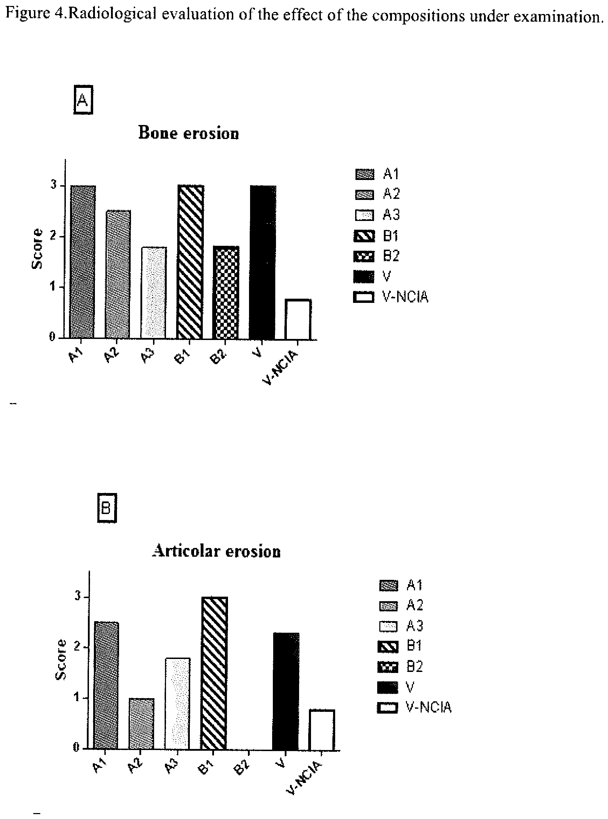

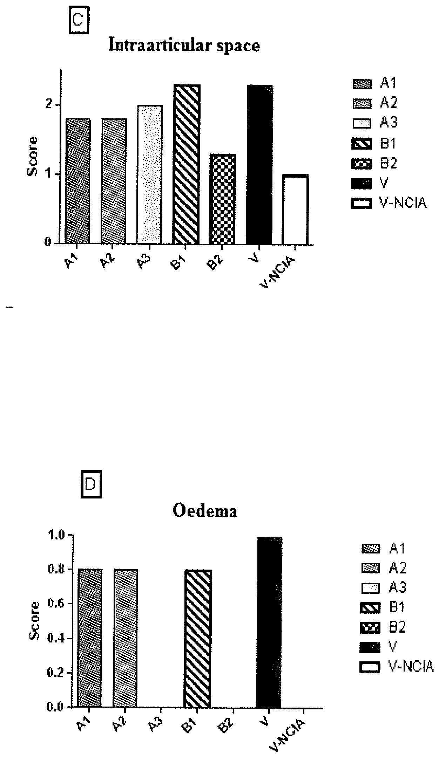

The radiological analysis results are shown in Figure 4 below.

Both compositions, at the highest dose, are effective in reducing bone erosion by approximately 50% (Figure 4A).

Composition B (Group B2) resulted better than Composition A (Group A3) also with regards to alteration of intra-articular space (Figure 4C).

Finally, both of the compositions, at the higher dose alone, proved to be able to completely inhibit oedema development (Figure 4D).

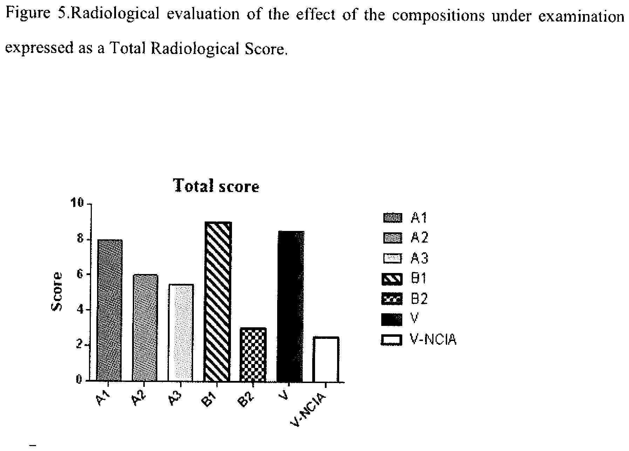

Both the radiological and the histological analyses confirm the greater effectiveness of Composition B with respect to Composition A, as proves clear from analysis of the Total Radiological Score shown in Figure 5.

Overall, the results obtained showed a powerful cartilage and bone damage-reducing effect in both Composition A and Composition B. In particular, Composition B, administered at the higher dose, proved to produce the maximum effect.

## Claims

1. Arginine as the only active principle for use in the treatment and/or prevention of arthrosis, **characterized in that** the daily dose thereof ranges from 400 mg to 2000 mg.

2. Arginine for use according to claim 1, **characterized in that** said arthrosis is a primary, secondary, localised and/or generalised arthrosis.

3. Arginine for use according to claim 1, **characterized in that** said it is administered orally.

4. Arginine for use according to claim 1, **characterized in that** it is administered topically.

5. Arginine for use according to claim 3, **characterized in that** it is administered in form of tablet, capsule, granule, granulate, powder, tablet, gel capsule, solution, suspension, emulsion, drops, syrup, powder for solutions, granules for solutions, granulate for suspensions, or powder for suspensions.

6. Arginine for use according to claim 4, **characterized in that** it is administered in form of cream, salve, ointment, gel, paste, milk, foam, medicated plaster, lotion, powder for topical application, solution, suspension, or emulsion.

7. Arginine for use according to any one of the previous claims, **characterized in that** it is administered to a human.

8. Arginine for use according to any one of the previous claims, **characterized in that** the daily dosage thereof ranges from 400 mg to 1600 mg.

9. Arginine for use according to any one of the previous claims, **characterized in that** it is used in the form of a pharmaceutically acceptable salt thereof, preferably hydrochloride salt.

## Patentansprüche

1. Arginin als einziges aktives Prinzip zur Verwendung bei der Behandlung und/oder Verhinderung von Arthrose, **dadurch gekennzeichnet, dass** die Tagesdosis von 400 mg bis 2000 mg reicht.

2. Arginin zur Verwendung nach Anspruch 1, **dadurch gekennzeichnet, dass** die Arthrose primäre, sekundäre, lokalisierte und/oder generalisierte Arthrose ist.

3. Arginin zur Verwendung nach Anspruch 1, **dadurch gekennzeichnet, dass** es oral verabreicht wird.

4. Arginin zur Verwendung nach Anspruch 1, **dadurch gekennzeichnet, dass** es topisch verabreicht wird.

5. Arginin zur Verwendung nach Anspruch 3, **dadurch gekennzeichnet, dass** es in Form einer Tablette, Kapsel, eines Granulats, Pulvers, einer Tablette, Gelkapsel, Lösung, Suspension, Emulsion, Tröpfchen, Syrup, Pulver für Lösungen, Granulate für Lösungen, Granulate für Suspensionen, oder Pulver für Suspensionen verabreicht wird.

6. Arginin zur Verwendung nach Anspruch 4, **dadurch gekennzeichnet, dass** es in Form von Creme, Salbe, Balsam, Gel, Paste, Milch, Schaum, medizinisches Pflaster, Lotion, Pulver für topische Anwendung, Lösung, Suspension oder Emulsion verabreicht wird.

7. Arginin zur Verwendung nach einem der vorstehenden Ansprüche, **dadurch gekennzeichnet, dass** es einem Menschen verabreicht wird.

8. Arginin zur Verwendung nach einem der vorstehenden Ansprüche, **dadurch gekennzeichnet, dass** die tägliche Dosis davon im Bereich von 400 mg bis 1600 mg ist.

9. Arginin zur Verwendung nach einem der vorstehenden Ansprüche, **dadurch gekennzeichnet, dass** es in Form eines pharmazeutisch annehmbaren Salzes davon, vorzugsweise des Hydrochloride-Salzes, verabreicht wird.

## Revendications

1. Arginine en tant que seul principe actif pour une utilisation dans le traitement et/ou la prévention de l'arthrose, **caractérisée en ce que** sa dose quotidienne est située dans la plage allant de 400 mg à 2000 mg.

2. Arginine pour une utilisation selon la revendication 1, **caractérisée en ce que** ladite arthrose est une arthrose primaire, secondaire, localisée et/ou généralisée.

3. Arginine pour une utilisation selon la revendication 1, **caractérisée en ce qu'**elle est administrée par voie orale.

4. Arginine pour une utilisation selon la revendication 1, **caractérisée en ce qu'**elle est administrée par voie topique.

5. Arginine pour une utilisation selon la revendication 3, **caractérisée en ce qu'**elle est administrée sous forme de comprimé, capsule, granule, granulat, poudre, comprimé, capsule en gel, solution, suspension, émulsion, gouttes, sirop, poudre pour solutions, granules pour solutions, granulat pour suspensions, ou poudre pour suspensions.

6. Arginine pour une utilisation selon la revendication 4, **caractérisée en ce qu'**elle est administrée sous forme de crème, onguent, pommade, gel, pâte, lait, mousse, emplâtre médical, lotion, poudre pour application topique, solution, suspension, ou émulsion.

7. Arginine pour une utilisation selon l'une quelconque des revendications précédentes, **caractérisée en ce qu'**elle est administrée à un humain.

8. Arginine pour une utilisation selon l'une quelconque des revendications précédentes, **caractérisée en ce que** sa dose quotidienne est située dans la plage allant de 400 mg à 1600 mg.

9. Arginine pour une utilisation selon l'une quelconque des revendications précédentes, **caractérisée en ce qu'**elle est utilisée sous la forme d'un sel pharmaceutiquement acceptable de celle-ci, de préférence du sel chlorhydrate.
